# EUROPÄISCHE PATENTSCHRIFT

(11) **EP 2 177 160 B1**
(45) Veröffentlichungstag und Bekanntmachung des Hinweises auf die Patenterteilung: **11.05.2011**
(21) Anmeldenummer: 08166803.0
(22) Anmeldetag: 16.10.2008
(51) Int. Cl.: A61B 5/15, A61B 5/00, G06F 19/00, G01N 33/48

(54) **Analysegerät mit benutzerfreundlicher Menüsteuerung**
Analysis device with user friendly menu control
Appareil d'analyse doté d'une commande de menu conviviale

(43) Veröffentlichungstag der Anmeldung: 21.04.2010
(73) Patentinhaber: Roche Diagnostics GmbH, 68305 Mannheim (DE); F. Hoffmann-La Roche AG, 4070 Basel (CH)
(72) Erfinder: Knoefel, Anja, 01099 Dresden (DE)
(74) Vertreter: Stößel, Matthias

(56) Entgegenhaltungen:
- EP-A- 1 338 295
- WO-A-2008/154312
- US-A1- 2007 093 786
- US-A1- 2007 179 358
- US-A1- 2008 114 299
- US-A1- 2008 171 922

## Beschreibung

Die Erfindung betrifft ein Analysegerät zum Nachweis eines Analyten in einer flüssigen Probe. Derartige Analysegeräte werden in verschiedenen Bereichen der Naturwissenschaft, Medizin und Technik eingesetzt, um verschiedene Arten von Analyten in verschiedenen Proben nachzuweisen. Ein Schwerpunkt der vorliegenden Anmeldung liegt auf Anwendungen im Bereich der Medizintechnik zum Nachweis eines oder mehrerer Analyten in einer Körperflüssigkeit, beispielsweise Blut, intestitieller Flüssigkeit oder Urin. Diese Analyten können beispielsweise Metaboliten umfassen. Beispiele derartiger Analyten sind Glucose, Laktat, Cholesterin oder ähnliche Stoffe und/oder Eigenschaften der Probe, beispielsweise Koagulationen. Die Erfindung ist jedoch nicht auf die genannten Anwendungen beschränkt.

In verschiedenen Bereichen der Naturwissenschaft, Medizin und Technik werden Analysegeräte eingesetzt. Ohne Beschränkung möglicher weiterer Ausgestaltungen des erfindungsgemäßen Analysegerätes wird im Nachfolgenden im Wesentlichen Bezug genommen auf Analysegeräte zum Nachweis von Blutglucose, insbesondere als Handgeräte ausgestaltete Analysegeräte.

Im Bereich der Gesundheitsvorsorge, Diagnostik oder Therapie ist es in vielen Fällen erforderlich, Körperfunktionen schnell und zuverlässig zu bestimmen. Ein typisches Beispiel ist die Diagnostik und Therapie von Blutzuckererkrankungen, wie Diabetes vom Typ I oder Typ II. Diabetiker müssen in der Regel ihre Blutzuckerkonzentration mindestens einmal oder typischerweise bis zu sieben Mal täglich schnell und sicher bestimmen. Um den Tagesablauf des Diabetikers nicht unnötig einzuschränken, wurden daher Analysegeräte entwickelt, welche mobil sind und den qualitativen und/oder quantitativen Nachweis des mindestens einen Analyten, insbesondere Blutglucose, auch in der Freizeit, am Arbeitsplatz oder an sonstigen Orten schnell und zuverlässig ermöglichen. Auf derartige bekannte Analysegeräte kann auch im Rahmen der vorliegenden Erfindung Bezug genommen werden.

Der qualitative und/oder quantitative Nachweis des mindestens einen Analyten erfolgt dabei beispielsweise auf optischem und/oder elektrochemischem Wege. So können beispielsweise Testelemente eingesetzt werden, welche mindestens ein Nachweismaterial umfassen, das bei Kontakt mit dem nachzuweisenden Analyten spezifisch und messbar mindestens eine physikalische und/oder chemische Eigenschaft ändert. Beispielsweise können auf diese Weise optisch und/oder elektrochemisch Analyte wie Blutglucose nachgewiesen werden. Die Testelemente können dabei in unterschiedlichster Form als einzelne Testelemente und/oder als Testelemente für den mehrfachen Gebrauch ausgestaltet sein. Es sind beispielsweise Einzelteststreifen, Mehrfachteststreifen, Testbänder mit einem oder mehreren Testfeldern, faltbare Testelemente, Teströhrchen, Testscheiben mit einem oder mehreren Testfeldern oder andere Ausführungsformen bekannt. Grundsätzlich sind im Rahmen der vorliegenden Erfindung sämtliche Arten bekannter Testelemente einsetzbar.

Analysegeräte weisen in der Regel mindestens eine Steuerung auf, welche eine, mehrere oder alle Funktionen des Analysegerätes steuern kann. Diese Steuerung kann beispielsweise den qualitativen und/oder quantitativen Nachweis des mindestens einen Analyten steuern. Zu diesem Zweck können beispielsweise Messsignale ausgewertet werden, Messdaten gespeichert werden, Messdaten ausgewertet werden, Messdaten verwaltet werden, Messdaten an andere Geräte übermittelt werden, Messdaten für einen Benutzer optisch und/oder akustisch und/oder haptisch dargestellt werden oder ähnliche Funktionen durchgeführt werden. Beispielsweise können derartige Steuerungen ein oder mehrere Datenverarbeitungsgeräte umfassen sowie ggf. einen oder mehrere nichtflüchtige und/oder flüchtige Datenspeicher.

Analysegeräte und/oder deren Steuerungen weisen in der Regel eine Menüsteuerung auf, welche beispielsweise ganz oder teilweise durch eine entsprechende Software implementiert sein kann. Eine derartige Menüsteuerung kann einem Benutzer beispielsweise eine Kontrolle und/oder Benutzung des Analysegerätes ermöglichen. So kann die Menüsteuerung beispielsweise einen Zugriff bzw. eine Steuerung der Datenspeicherung ermöglichen und/oder eine Auswertung und/oder Darstellung von Messdaten.

Die Ausgestaltung der Menüsteuerung stellt dabei technisch eine erhebliche Herausforderung dar. So sind insbesondere Blutzuckermessgeräte für einen weiten Kreis von Anwendern vorgesehen, unter welchen sich auch Kinder oder ältere Patienten befinden können. Analysegeräte, welche in der Regel unter erheblichem Kostendruck und möglichst bauraumsparend hergestellt werden müssen, weisen jedoch üblicherweise lediglich zwei oder drei Bedienelemente auf, welche dem Benutzer für eine Menüsteuerung zur Verfügung stehen. Aufgrund der reduzierten Zahl der Bedienelemente muss sich ein Benutzer häufig umständlich entlang einer Menüstruktur der Menüsteuerung durcharbeiten, um bestimmte Funktionen im Analysegerät auszuwählen und/oder ansteuern zu können. Gerade Kindern oder älteren Patienten bereitet eine derartige umständliche Menüsteuerung jedoch teilweise erheblich Probleme, da beispielsweise der Umgang mit lediglich auf einem Display dargestellten Menüoptionen und die Auswahl einer oder mehrerer dieser Funktionen für viele Patienten der genannten Zielgruppen ungewohnt ist.

Eine weitere Herausforderung derartiger Menüsteuerungen besteht darin, dass Anzeigenelemente wie Displays derartiger Analysegeräte sehr flexibel hinsichtlich der Darstellungsoptionen ausgestaltet werden müssen, um die Menüoptionen bei der Menüsteuerung verständlich darzustellen. So sollte sich, auch ohne dass ein Benutzerhandbuch zu Rate gezogen wird, aus den angezeigten Informationen dem Benutzer die Menüsteuerung vollständig erschließen. Insbesondere sollten sich die in einer bestimmten Menüebene aufgeführten Funktionen und/oder Parameter für den Benutzer erkennen lassen, ohne dass zusätzliche Informationen und/oder Hintergrundwissen erforderlich sind.

Diese verständliche Anzeige von Informationen, welche sehr unterschiedlicher Art und veränderlich sein können, erfordert jedoch in aller Regel aufwendig gestaltete Anzeigenelemente. So lassen sich beispielsweise einfache segmentiere Displays, wie beispielsweise die bekannten Sieben-Segment-Anzeigen, häufig in Analysegeräten der beschriebenen Art nicht oder nur eingeschränkt einsetzen, da die Flexibilität derartiger Displays und/oder die Art der mittels dieser Displays anzeigbaren Informationen äußerst eingeschränkt ist. Gerade derartige segmentierte Displays sind jedoch in vielen Fällen wünschenswert und anderen Arten von Displays, beispielsweise pixelierten Displays, vorzuziehen. So sind derartige Displays in der Regel hinsichtlich ihres Ablesewinkels und/oder hinsichtlich ihrer Erkennbarkeit besser ablesbar, was gerade für ältere Patienten einen erheblichen Vorteil darstellt. Ein weiterer Aspekt liegt darin, dass segmentierte Displays im Gegensatz zu pixelierten Displays eine erheblich einfachere elektronische Ansteuerung erfordern und in der Regel auch einen geringeren Energiebedarf und Bauraumbedarf aufweisen. Auch Kostenaspekte spielen eine erhebliche Rolle, da Analysegeräte für den Einsatz im privaten Bereich in der Regel unter erheblichem Kostendruck hergestellt werden.

Aus anderen Bereichen der Medizintechnik sind Geräte bekannt, welche ebenfalls eine Bedienung durch einen Benutzer erfordern. So beschreibt beispielsweise US 6,585,698 B1 einen Medikationsstift mit einem Gehäuse und einem Aktor zum Einstellen und Bereitstellen einer Dosis eines Medikaments. Mit dem Aktor ist ein Prozessor gekoppelt, um einen Wert zu bestimmen, der einer durch den Aktor eingestellten Dosis entspricht. Die vom Prozessor bereitgestellte Information wird auf einem Display dargestellt. Der Aktor weist dabei zwei Zustände auf. In einem ersten Zustand koppelt der Aktor mit einem Antriebsmechanismus, um die Medikation bereitzustellen. In einem zweiten Zustand ist der Aktor von dem Antriebsmechanismus abgekoppelt und dient als ein vom Benutzer einstellbares Eingabeelement zum Ändern und Einstellen von Parametern in mindestens einem voreingestellten Modus des Medikationsstiftes.

Auch die in US 6,585,698 B1 oder in ähnlichen, Medikationsstifte betreffenden Schriften dargestellten Konstruktionen, welche eine Eingabe von Parametern ermöglichen, lösen die oben dargestellte Problematik jedoch in der Regel nicht. So ist die Informationsfülle der auf dem Display derartiger Medikationsstifte darzustellenden Informationen üblicherweise nicht vergleichbar mit den komplexen und umfangreichen Informationen eines Analysegerätes. Auch ist die Menüsteuerung derartiger Medikationsstifte mit der komplexen Menüsteuerung von Analysegeräten der Regel nicht vergleichbar, da sich derartige Menüsteuerungen zumeist lediglich auf die Auswahl einer bereitzustellenden Dosis beschränken. Zudem ist auch bei den beschriebenen Medikationsstiften, sollen mehrere Menüebenen verwaltet werden, nach wie vor eine komplexe Führung durch diese Menüebenen erforderlich. Dokument US 2008/171922 A1 offenbart ein Analysegerät mit einem mechanisch verstellbaren Auswahlelement, welches in verschiedene Auswahlpositionen einstellbar ist und diese nach dem Einstellen beibehält. Durch das Auswahlelement wird ein direktes Springen zu vorgegebenen Menüpunkten einer Menüsteuerung und deren Auswahl ermöglicht. Die Menüführung wird jedoch über eine vergleichsweise hochauflösende Flüssigkristallanzeige erläutert, welches die Menüpunkte oder deren Inhalte bezeichnet, so dass die oben beschriebene Problematik bekannter Analysegeräte erhalten bleibt.

Es ist daher eine Aufgabe der vorliegenden Erfindung, ein Analysegerät bereitzustellen, welches die Nachteile bekannter Analysegeräte vermeidet. Insbesondere soll das Analysegerät eine benutzerfreundliche Menüsteuerung bei gleichzeitig geringem technischem Aufwand ermöglichen.

Diese Aufgabe wird durch ein Analysegerät mit den Merkmalen des unabhängigen Anspruchs gelöst. Vorteilhafte Weiterbildungen der Erfindung, welche einzeln oder in Kombination realisierbar sind, sind in den abhängigen Ansprüchen dargestellt.

Die Erfindung betrifft ein Analysegerät zum Nachweis eines Analyten in einer flüssigen Probe. Der Nachweis kann dabei qualitativ und/oder quantitativ erfolgen. Bezüglich der möglichen nachzuweisenden Analyten und/oder bezüglich der möglichen Ausgestaltungen der flüssigen Probe, der möglichen Nachweismethoden und/oder dem möglichen Einsatz von bestimmten Testelementen kann beispielsweise auf die obige Beschreibung des Standes der Technik verwiesen werden. Das Analysegerät kann insbesondere als tragbares Handgerät ausgestaltet sein und eingerichtet sein, um von einem Benutzer ohne größeren Aufwand beispielsweise in einer Tasche eines Kleidungsstückes mitgeführt zu werden. Im Folgenden wird das Analysegerät insbesondere unter Bezugnahme auf ein Analysegerät zum Nachweis von Glucose in einer Körperflüssigkeit, insbesondere zum Nachweis von Blutglucose, beschrieben. Alternativ zu dieser bevorzugten Ausgestaltung sind jedoch grundsätzlich auch andere Ausgestaltungen möglich.

Das Analysegerät umfasst mindestens eine Steuerung, welche beispielsweise gemäß der oben dargestellten Beschreibung bekannter Steuerungen ausgestaltet sein kann. Die Steuerung kann insbesondere ein oder mehrere Datenverarbeitungsgeräte umfassen, welche beispielsweise softwaretechnisch eingerichtet sein können. Die Steuerung ist eingerichtet, um ein Funktionsmenü mit mindestens zwei Menüpunkten bereitzustellen. Unter einem Funktionsmenü wird dabei eine zumindest teilweise softwaretechnisch ausgestaltete Einrichtung der Steuerung verstanden, welche einem Benutzer eine Interaktion mit dem Analysegerät bietet. So kann beispielsweise auf die eingangs beschriebenen Beispiele derartiger Funktionsmenüs verwiesen werden. Über dieses Funktionsmenü können somit beispielsweise Steuerbefehle eingegeben werden, Messungen initiiert werden, Messergebnisse abgefragt werden, beispielsweise aktuelle Messergebnisse und/oder gespeicherte Messergebnisse, es kann eine Verwaltung gespeicherter Daten vorgenommen werden, es kann eine akustische, optische, haptische oder elektronische Ausgabe von Daten angefordert werden, es kann eine Auswertung von Messergebnissen initiiert werden, es können bestimmte Werte (Parameter, Variablen) eingestellt werden, wie beispielsweise Uhrzeit, Datum, Benutzer oder ähnliches, oder es können Kombinationen der genannten und/oder anderer Aktionen durchgeführt werden. Verschiedene andere Ausgestaltungen sind möglich.

Das Funktionsmenü weist mindestens zwei Menüpunkte auf, vorzugsweise drei, vier oder mehr Menüpunkte. Unter einem Menüpunkt wird dabei jeweils ein bestimmter Modus einer Interaktion zwischen dem Analysegerät und dem Benutzer verstanden. So kann jeder Menüpunkt dem Benutzer die Eingabe unterschiedlicher Arten von Befehlen und/oder Informationen ermöglichen, oder jeder Menüpunkt kann unterschiedliche Arten von Informationen an den Benutzer bereitstellen. Ein derartiger Menüpunkt kann dabei auch in Unter-Menüpunkte aufgeteilt werden, so dass eine oder mehrere Menüebenen in jedem Menüpunkt vorgesehen sein können. In diesem Fall kann das Funktionsmenü mit seinem Menüpunkten beispielsweise ganz oder teilweise als Baum-Struktur ausgestaltet sein.

Weiterhin umfasst das Analysegerät mindestens ein mechanisch verstellbares Auswahlelement. Unter einem Auswahlelement ist dabei ein Element zu verstehen, welches durch eine Einwirkung eines Benutzers, insbesondere eine manuelle Einwirkung, beispielsweise ein Verdrehen und/oder Verschieben in mindestens zwei verschiedenen Positionen und/oder Orientierungen positionierbar bzw. einstellbar ist, vorzugsweise in drei, vier oder mehr verschiedene Positionen bzw. Orientierungen. Dabei können stufenlos verstellbare Auswahlelemente eingesetzt werden, wobei jedoch die Verwendung von nicht-stufenlosen Auswahlelementen, also Auswahlelementen, welche jeweils in einer bestimmten Position und/oder Orientierung fühlbar einrasten, bevorzugt ist.

Das Auswahlelement ist eingerichtet, um nach dem Einstellen in der eingestellten Auswahlposition zu verbleiben. Dies bedeutet, dass ohne weitere Einwirkung von außen, beispielsweise durch einen Benutzer, die eingestellte Auswahlposition, also die eingestellte Position und/oder Orientierung, zumindest näherungsweise erhalten bleibt. Unter "zumindest näherungsweise" ist dabei vorzugsweise ein exaktes Verbleiben in der eingestellten Auswahlposition zu verstehen. Grundsätzlich sind jedoch auch leichte Abweichungen von der ursprünglich eingestellten Auswahlposition zu verstehen, beispielsweise bedingt durch Federkräfte oder beispielsweise in Form eines Springens in eine nächstgelegene vorgegebene Auswahlposition, beispielsweise wenn das Auswahlelement nicht stufenlos verstellbar ist und die vom Benutzer eingestellte Auswahlposition nicht exakt den möglichen Auswahlpositionen entspricht. Insgesamt steht somit das Auswahlelement beispielsweise im Gegensatz zu üblicherweise für eine Menüführung verwendeten Tastern oder Druckknöpfen, wie sie beispielsweise in herkömmlichen Blutglucosemessgeräten für eine Menüsteuerung verwendet werden. Die eingestellte Auswahlposition, also die eingestellte Position und/oder Orientierung, kann insbesondere für einen Betrachter von außen bei Betrachtung des Analysegeräts sichtbar und/oder erkennbar sein. Diese sichtbare, eingestellte Auswahlposition kann also vorzugsweise, beispielsweise ohne Zuhilfenahme weiterer, zusätzlicher Indikatoren, beispielsweise elektronischer Anzeigemittel, bereits selbst als Indikator für einen Benutzer dienen.

Das Auswahlelement soll durch einen Benutzer in mindestens zwei, vorzugsweise drei, vier oder mehr verschiedene Auswahlpositionen einstellbar sein. Unter einer Auswahlposition kann dabei eine Orientierung und/oder Positionierung des Auswahlelementes zu verstehen sein. Beispielsweise können dies bestimmte Drehwinkelpositionen eines Drehknopfes und/oder bestimmte Schiebepositionen eines Schiebers sein. Wie oben dargestellt, können diese Auswahlpositionen beispielsweise haptisch fühlbar ausgestaltet sein, so dass ein Benutzer erkennt, dass eine Auswahlposition erreicht ist.

Die Steuerung soll dabei eingerichtet sein, um entsprechend der eingestellten Auswahlposition einen Menüpunkt auszuwählen. So kann beispielsweise für einen Menüpunkt, für mehrere Menüpunkte oder für alle Menüpunkte des oben beschriebenen Funktionsmenüs jeweils eine bestimmte Auswahlposition vorgegeben sein. Das Auswahlelement ermöglicht es einem Benutzer somit, vorzugsweise ohne dass eine komplexe Software-Menüsteuerung vorgesehen ist, unmittelbar zu bestimmten Menüpunkten des Funktionsmenüs zu springen, ohne sich durch ein aufwendiges Software-Menü hindurch zu arbeiten. Die Menüpunkte könnten damit die End-Menüpunkte in einer Baum-Struktur sein. Alternativ oder zusätzlich können jedoch auch ein, mehrere oder alle Menüpunkte in Unter-Menüpunkte aufgeteilt sein, welche beispielsweise zusätzlich durch eine Software-Menüsteuerung oder, alternativ oder zusätzlich, durch ein oder mehrere weitere Auswahlelemente der beschriebenen Art erreichbar sein können.

Weiterhin umfasst das Analysegerät mindestens ein Bedienelement. Das mindestens eine Bedienelement kann von dem mindestens einen Auswahlelement ganz oder teilweise getrennt ausgestaltet sein. Alternativ kann das mindestens eine Bedienelement jedoch auch beispielsweise ganz oder teilweise mit dem mindestens einen Auswahlelement zusammengefasst werden, beispielsweise indem auf einem als Drehknopf ausgestalteten Auswahlelement ein, zwei oder mehr Bedienelemente angeordnet sind. Das mindestens eine Bedienelement kann dabei auch einen oder mehrere Taster umfassen und/oder andere Arten von Bedienelementen, welche nach einer Betätigung wieder in ihre Ausgangsposition zurückkehren, im Gegensatz zur Ausgestaltung des mindestens einen Auswahlelements.

Das Analysegerät, insbesondere die Steuerung, insbesondere das Funktionsmenü, sollen derart eingerichtet sein, dass mittels dieses Bedienelements mindestens ein Parameter in dem durch die eingestellte Auswahlposition ausgewählten Menüpunkt durch den Benutzer beeinflussbar ist. Unter einem Parameter kann dabei eine beliebige Information und/oder ein beliebiger Befehl zu verstehen sein, welcher von dem Benutzer an das Analysegerät, insbesondere die Steuerung, insbesondere das Funktionsmenü, übermittelt werden soll. Beispielsweise kann es sich bei diesem mindestens einen Parameter und der darin enthaltenen Information um einen numerischen Wert handeln, beispielsweise eine Ziffer einer einzustellenden Uhrzeit, eines Datums, oder ähnliches. Ist mittels des Auswahlelementes beispielsweise eine bestimmte Stelle in einer Zahl ausgewählt, was ebenfalls unter einem bestimmten Menüpunkt verstanden werden kann, so kann mittels des Bedienelementes diese Stelle beispielsweise auf einfache Weise mittels Erhöhungs- und/oder Erniedrigungstasten in ihrem numerischen Wert erhöht und/oder erniedrigt werden. Alternativ oder zusätzlich kann, beispielsweise zur Übermittlung von Steuerbefehlen, der Menüpunkt auch eine Auswahl von Steuerbefehlen umfassen, welche nach Anwählen des Menüpunktes mittels des Auswahlelementes auf einfache Weise, beispielsweise ebenfalls mittels Auswahltasten des Bedienelementes, auswählbar sind. Insofern ist der Begriff des Parameters vorliegend weit zu fassen.

Dabei kann die Menüsteuerung derart ausgestaltet sein, dass lediglich einer der vorgesehenen und auswählbaren Menüpunkte, einige dieser Menüpunkte oder alle dieser Menüpunkte die Beeinflussung eines oder mehrerer Parameter erforderlich machen und/oder ermöglichen. Dementsprechend kann diese Beeinflussung des mindestens einen Parameters durch das mindestens eine Bedienelement in einem, mehreren oder allen Menüpunkten möglich sein, was im Rahmen der vorliegenden Erfindung mit umfasst sein soll.

Das Bedienelement kann dabei auf verschiedene Weise ausgestaltet sein und kann eine oder mehrere Funktionstasten und/oder andere Arten von Eingabeelementen umfassen, welche eine einfache Interaktion mit dem Analysegerät ermöglichen und welche vorzugsweise multifunktional ausgestaltet sind. Vorzugsweise sind nicht mehr als drei Bedienelemente vorgesehen, beispielsweise ein Bedienelement zum Erhöhen eines Parameters und/oder um einen Schritt in einer Auswahl voranzugehen, ein Bedienelement zum Erniedrigen eines Parameters und/oder um einen Schritt in einer Auswahl zurückzugehen, und ein Bedienelement für eine Eingabe, eine Bestätigung oder ähnliches. Verschiedene Ausgestaltungen sind denkbar. Und verschiedene mögliche Ausführungsbeispiele werden unten näher beschrieben.

Die erfindungsgemäße Ausgestaltung des Analysegerätes mit dem Auswahlelement, welches ein direktes Springen zu vorgegebenen Menüpunkten ermöglicht, bewirkt eine Vereinfachung der Menüsteuerung bei gleichzeitiger einfacher Ausgestaltung des Analysegerätes. So wird eine gezielte Menüsteuerung für den Benutzer erleichtert, indem der Benutzer unmittelbar über das Auswahlelement, beispielsweise einen mechanisch verstellbaren Knopf, einen bestimmten Menüpunkt auswählt. So kann, in Abhängigkeit von einer Stellung des Knopfes, eine direkte Ansteuerung eines bestimmten Menüpunktes und somit einer Menüebene gegeben sein.

Das Analysegerät weist ein Gehäuse auf und Kann beispielsweise eine Markierung und oder Beschriftung aufweisen, welche unmittelbar und vorzugsweise unabhängig von einer elektrischen Funktionalität des Analysegerätes die Menüsteuerung erläutert. So kann diese Beschriftung beispielsweise die jeweiligen Menüpunkte auflisten und eine selbsterklärende Bedienung für einen Benutzer ermöglichen. Sobald das Auswahlelement, beispielsweise der Knopf, entsprechend zu dem aufgelisteten Menüpunkt positioniert ist, kann eine direkte Ansteuerung dieses Menüpunktes und/oder der Menüebene erfolgen und eine entsprechende Eingabe und/oder Auswahl von Daten kann dann entsprechend über das mindestens eine Bedienelement erfolgen.

Auf dem Gehäuse kann eine für den Benutzer wahrnehmbare Markierung vorgesehen sein, beispielsweise eine optisch wahrnehmbare und/oder haptisch wahrnehmbare Markierung. Das Auswahlelement kann dann relativ zu der Markierung in seiner Position und/oder Orientierung verstellbar sein. Die Markierung ermöglicht es also dem Benutzer, jeweils die eingestellte Auswahlposition zu erkennen. Alternativ oder zusätzlich zu einer Markierung auf dem Gehäuse kann auch eine entsprechende Markierung auf dem Auswahlelement vorgesehen sein. Aus der relativen Stellung dieser Markierungen kann dann der Benutzer die eingestellte Auswahlposition erkennen.

Die Markierung und das Bedienelement können dabei auch zumindest teilweise zusammengefasst sein. So kann beispielsweise das Bedienelement in einer festen Positionierung und/oder Orientierung auf und/oder in dem Gehäuse angeordnet sein und somit ganz oder teilweise Bestandteil der Markierung sein. Das Auswahlelement kann dann relativ zu diesem Bedienelement positioniert und/oder orientiert werden. Alternativ oder zusätzlich kann das mindestens eine Bedienelement jedoch auch ganz oder teilweise in dem Auswahlelement selbst integriert sein, so dass die Position und/oder Orientierung des Bedienelements relativ zum übrigen Gehäuse sich mit einer Veränderung der eingestellten Auswahlposition ebenfalls ändert. In diesem Fall kann das Bedienelement beispielsweise Bestandteil einer auf dem Auswahlelement angeordneten Markierung sein.

Das Gehäuse und/oder das Auswahlelement weisen mindestens eine Funktionsmarkierung auf. Diese Funktionsmarkierung, welche die oben dargestellte Beschriftung ganz oder teilweise umfasst ist eingerichtet, um mindestens einen der Menüpunkte und/oder mindestens einen Inhalt mindestens eines der Menüpunkte zu bezeichnen. Dabei kann die Bezeichnung der Funktionsmarkierung beispielsweise lediglich eine einfache Benennung des Menüpunkts umfassen, beispielsweise "Messwerte anzeigen", "Datum einstellen", "Uhrzeit einstellen", oder ähnliche, vorzugsweise für einen Benutzer selbsterklärend verständliche Bezeichnungen. Alternativ oder zusätzlich kann die Funktionsmarkierung jedoch weitere Informationen enthalten, beispielsweise eine vollständige oder teilweise der mittels des Bedienelements in der jeweiligen Auswahlposition bzw. den jeweils eingestellten Menüpunkt beeinflussbare Parameter. Eine derartige Anzeige erleichtert die Benutzerführung zusätzlich. Diese Darstellung verringert zudem die Zahl der Informationen, welche ggf. auf einem Anzeigenelement wie beispielsweise einem Display anzuzeigen sind, so dass dieses Anzeigenelement, welches optional vorgesehen sein kann, stark vereinfacht ausgestaltet werden kann. Zudem sind für einen Benutzer unmittelbar die beeinflussbaren Parameter erkennbar, welche ansonsten in einer Menüsteuerung lediglich aufgrund von Hintergrundwisscn und/oder durch umständliches Ausprobieren erkennbar wären. Sowohl in technischer Hinsicht als auch hinsichtlich einer Benutzerfreundlichkeit weist eine derartige Ausgestaltung somit erhebliche Vorteile auf.

Wie oben dargestellt, kann das Auswahlelement auf verschiedene Weisen ausgestaltet sein. Besonders bevorzugt ist es, wenn das Auswahlelement mindestens einen Drehschalter, beispielsweise einen Knopf, insbesondere einen Drehknopf, und/oder einen Schiebeschalter aufweist. Wie oben dargestellt, können derartige Schalter stufenlos oder vorzugsweise stufenweise verstellbar sein.

Besonders bevorzugt im Rahmen der vorliegenden Erfindung ist es, wenn das Auswahlelement ganz oder teilweise in das Gehäuse integriert ist. Auf diese Weise kann das Auswahlelement die äußere Formgebung und damit Handhabbarkeit des Gehäuses im Wesentlichen unbeeinflusst lassen. So kann das Gehäuse beispielsweise zumindest teilweise im Wesentlichen zylinderförmig ausgestaltet sein. Unter "im Wesentlichen zylinderförmig" können dabei auch leichte Abweichungen von der Zylinderform toleriert werden. Bei dieser zylinderförmigen Ausgestaltung kann das Auswahlelement mindestens einen das Gehäuse zumindest teilweise umschließenden Drehring umfassen, beispielsweise einen im Wesentlichen zylinderringförmigen Drehring, welcher beispielsweise parallel zur Achse der Zylinderform des Gehäuses orientiert sein kann. Dieser Drehring kann somit Teil der Zylinderform des Gehäuses sein. Der Drehring ist dabei zur Einstellung der Auswahlposition relativ zum übrigen Gehäuse verdrehbar.

Wie oben dargestellt, kann das Bedienelement zumindest teilweise in dem Auswahlelemcnt und/oder in dem übrigen Gehäuse integriert sein. Vorzugsweise sind nicht mehr als drei Bedienelemente vorgesehen, da die Möglichkeit der unmittelbaren Auswahl von Menüpunkten durch das Auswahlelement dennoch eine einfache Menüsteuerung gewährleisten kann. Auf diese Weise lassen sich Kosten, welche durch zusätzliche Bedienelemente entstehen, einsparen, bei gleichzeitig einfacher Menüsteuerung durch das mindestens eine, vorzugsweise unabhängig von der Bedienung des Bedienelementes bedienbare Auswahlelement.

Das Analysegerät kann weiterhin mindestens ein Anzeigenelement zur Anzeige mindestens einer veränderlichen Information umfassen. Ein derartiges Anzeigenelement, insbesondere ein Display, kann dabei einfach gestaltet werden, da die Menüsteuerung im Wesentlichen durch das Auswahlelement und ggf. durch eine Markierung und/oder Beschriftung erfolgen kann. Insofern ist die Anzahl und Variabilität der darzustellenden Informationen auf ein Minimum reduzierbar.

Diese Möglichkeit, das Anzeigenelement einfach auszugestalten, ermöglicht beispielsweise die Verwendung eines oder mehrerer segmentierter Displays. Das Anzeigenelement kann dementsprechend mindestens ein segmentiertes Display, beispielsweise ein Sieben-Segment-Dispaly, umfassen. Die Vorteile derartiger ausgestalteter segmentierter Displays wurden eingangs bereits erwähnt und sind insbesondere in der Ablesbarkeit, der kostengünstigen Ausgestaltung möglicher Treiberschaltungen, dem geringen Bauraum und dem geringen Energiebedarf zu sehen.

Das Anzeigenelement kann beispielsweise ganz oder teilweise in einem Deckel eines Gehäuses des Analysegerätes integriert sein. Bei einer zylindrischen Ausgestaltung des Gehäuses kann dieser Deckel beispielsweise einen zylindrischen Deckel des Analysegerätes umfassen. Der Deckel kann auch aufschwenkbar und/oder aufklappbar ausgestaltet sein, um mindestens ein innenliegendes Element des Analysegerätes freizugeben. Beispielsweise kann der Deckel aufklappbar oder aufschwenkbar sein, um eine Applikationsposition zur Aufgabe der flüssigen Probe und/oder ein Testelement freizugeben. Auf diese Weise können die Testelemente und/oder die Applikationsposition geschützt im Inneren des Gehäuses des Analysegerätes angeordnet sein und erst durch Aufklappen und/oder Aufschwenken des Deckels freigelegt werden. Die Kombination des Anzeigenelementes mit dem aufschwenkbaren und/oder aufklappbaren Deckel bewirkt eine zusätzliche Bauraumersparnis.

Alternativ oder zusätzlich kann auch der Deckel ganz oder teilweise drehbar ausgestaltet sein. Auf diese Weise kann beispielsweise das Auswahlelement ganz oder teilweise in dem drehbaren Deckel integriert sein. Beispielsweise kann das Auswahlelement in diesem Fall einen Drehschalter umfassen, welcher mit Drehung des Deckels betätigt werden kann.

Neben den genannten Elementen kann das Analysegerät weitere Funktionen und/oder Elemente umfassen. So kann beispielsweise, zusätzlich zu dem oben dargestellten qualitativen und/oder quantitativen Nachweis des mindestens einen Analyten in der Probe, eine weitere medizinische Funktion gegeben sein, beispielsweise eine Probengewinnungsfunktion. Zu diesem Zweck kann das Analysegerät beispielsweise ein Probengewinnungselement umfassen, welches eingerichtet ist, um eine Hautpartie eines Benutzers zu perforieren. Derartige Probengewinnungselemente, welche mittels eines weitgehend schmerzfreien Durchstechens und/oder Durchschneidens der Hautpartie, beispielsweise im Bereich einer Fingerbeere und/oder eines Ohrläppchens eines Patienten, eine Blutprobe und/oder eine andere Probe von Körperflüssigkeit erzeugen, werden häufig auch als Stechhilfen bezeichnet. Sie enthalten in der Regel ein oder mehrere Lanzettenelemente, mittels derer die Perforation der Hautpartie zu gewährleisten ist. Das Probengewinnungselement kann von dem übrigen Analysegerät reversibel abkoppelbar und separat verwendbar sein. Im gekoppelten Zustand bilden dann das Probengewinnungselement und das übrige Analysegerät eine Einheit, welche einfach und leicht zu transportieren ist. Im abgekoppelten Zustand kann das Probengewinnungselement dann separat gehandhabt werden, um beispielsweise eine flüssige Probe zu generieren. Alternativ sind jedoch auch fest in das übrige Analysegerät integrierte Probengewinnungselemente möglich.

### Ausführungsbeispiele

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind figurenschematisch dargestellt. Gleiche Bezugsziffern in den einzelnen Figuren bezeichnen dabei gleiche oder funktionsgleiche bzw. hinsichtlich ihrer Funktionen entsprechende Elemente.

Im Einzelnen zeigt:
- Figur 1: eine Seitenansicht eines ersten Ausführungsbeispiels eines erfindungsgemä- ßen Analysegerätes;
- Figur 2: eine Detaildarstellung eines Auswahlelementes und der Bedienelemente des Analysegerätes gemäß Figur 1;
- Figur 3: eine Draufsicht auf einen ein Display enthaltenden Deckel des Analysegerä- tes gemäß Figur 3;
- Figur 4: ein weiteres Ausführungsbeispiel eines erfindungsgemäßen Analysegerätes in einer zu Figur 2 analogen Darstellung;
- Figur 5: ein weiteres Ausführungsbeispiel eines erfindungsgemäßen Analysegerätes in einer zu Figur 2 analogen Darstellung;
- Figur 6: eine Schnittdarstellung eines möglichen Ausführungsbeispiels eines Analy- segerätes; und
- Figur 7: eine schematische Darstellung eines Aufschwenkens eines Deckels eines erfindungsgemäßen Analysegerätes.

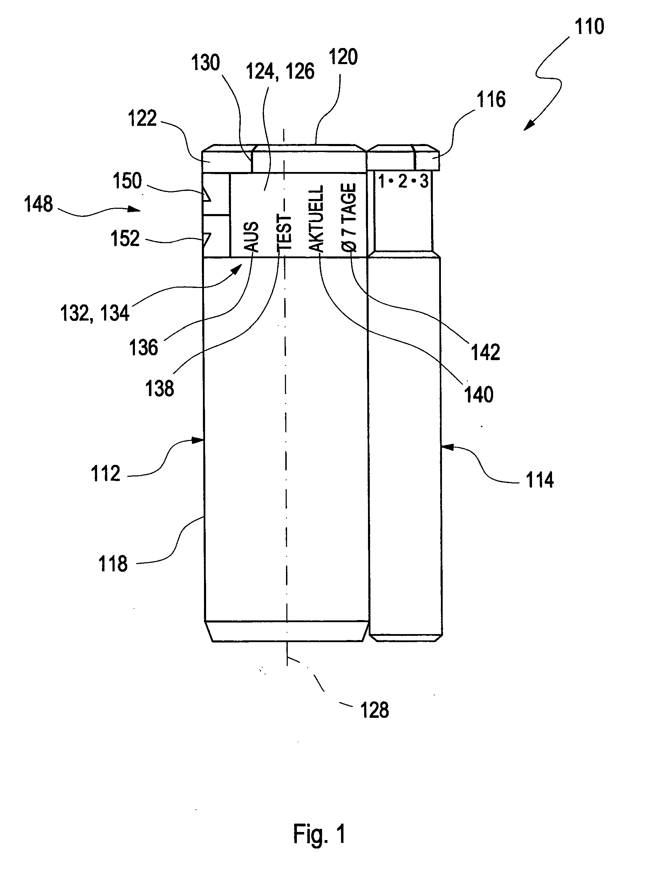

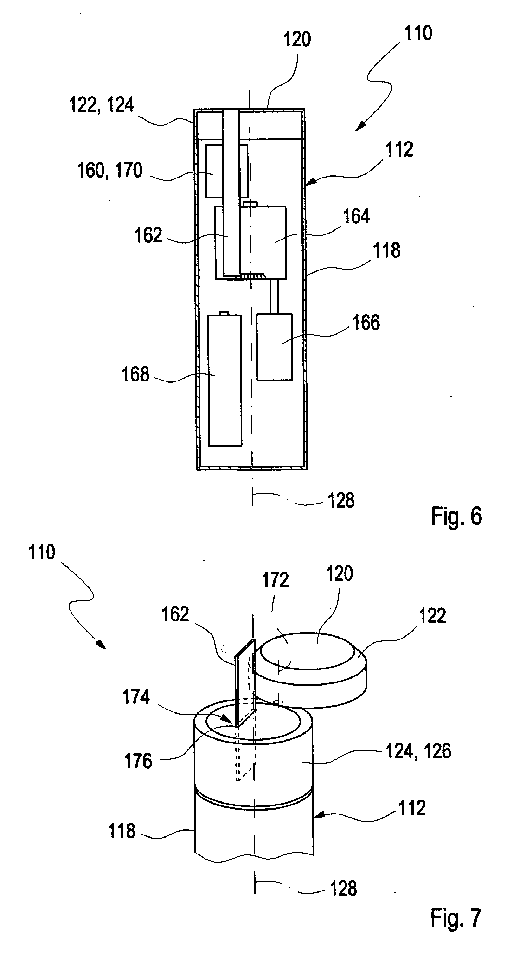

In Figur 1 ist ein erstes Ausführungsbeispiel eines erfindungsgemäßen Analysegerätes 110 dargestellt. Dieses Analysegerät 110 kann beispielsweise als Analysegerät zur Blutzuckerbestimmung ausgestaltet sein und kann mittels einem oder mehreren Testelementen eine Blutzuckerkonzentration 'bestimmen. Die Testelemente sowie eine entsprechende Vorrichtung zur Wechselwirkung des Analysegerätes 110 mit den Testelementen sind in Figur 1 nicht dargestellt. Beispielsweise können auf einer Oberseite und/oder einer Unterseite des Analysegerätes 110 gemäß Figur 1 entsprechende Öffnungen vorgesehen sein. Alternativ oder zusätzlich kann das Analysegerät 110 auch an einer oder mehreren Stellen geöffnet werden, um eine Applikation einer Blutprobe und/oder ein Einbringen eines Testelementes oder ein Freigeben einer Applikationsposition eines Testelementes zu ermöglichen. Diesbezüglich sei beispielsweise auf das unten beschriebene Ausführungsbeispiel gemäß Figur 7 verwiesen.

Das Analysegerät 110 ist in diesem Fall zweiteilig ausgestaltet und umfasst ein Messgerät 112, welches die eigentliche Funktionalität des Nachweises des Analyten bereitstellt. Daneben umfasst das Analysegerät 110 ein Probengewinnungselement 114, beispielsweise eine Stechhilfe. Dieses Probengewinnungselement 114 ist ebenfalls in Figur 1 lediglich symbolisch angedeutet und kann beispielsweise eingerichtet sein, um mittels eines Einstellknopfes 116 eine Einstechtiefe einer Lanzette einzustellen. Das Probengewinnungselement 114 ist mittels in Figur 1 nicht im Detail dargestellter Kopplungselemente vorzugsweise reversibel an das Messgerät 112 ankoppelbar. Zu diesem Zweck können beispielsweise Haken, Schienen, Bajonettverschlüsse oder ähnliche Koppelelemente vorgesehen sein. Auf diese Weise lässt sich das Probengewinnungselement 114 beispielsweise reversibel vom Messgerät 112 abkoppeln und unabhängig vom Messgerät 112 bedienen.

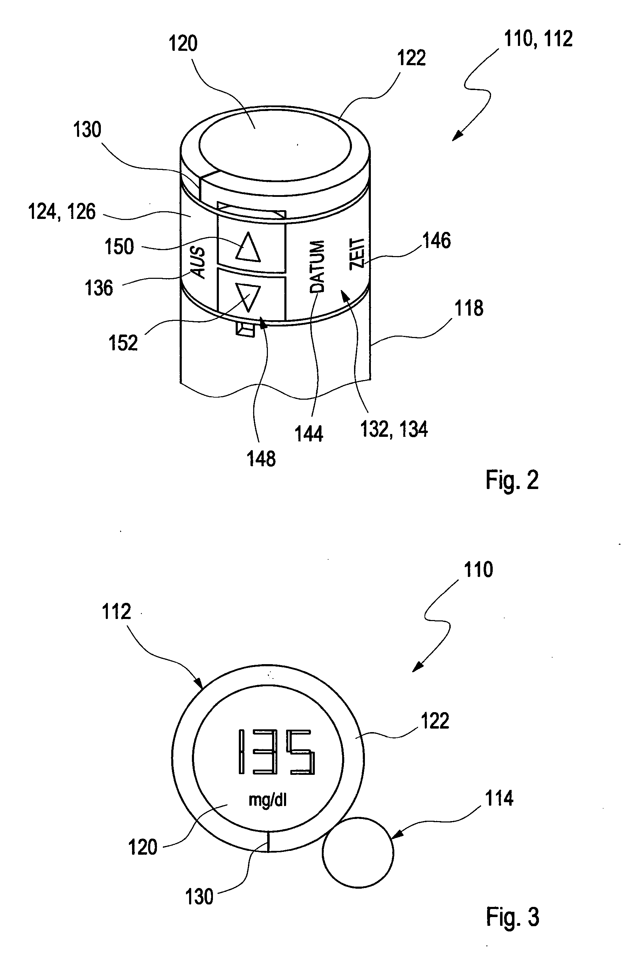

Das Analysegerät 110 bzw. das Messgerät 112 umfasst in dem dargestellten Ausführungsbeispiel ein Gehäuse 118, welches beispielsweise im Wesentlichen zylinderförmig ausgestaltet sein kann. In diesem Gehäuse können die entsprechenden Funktionselemente des Analysegerätes 110 bzw. des Messgerätes 112 angeordnet sein. Das Gehäuse 118 kann beispielsweise aus Kunststoff, aus Metall oder aus anderen Materialien hergestellt sein. Am oberen Ende des Gehäuses 118 ist ein Anzeigenelement 120 vorgesehen, welches beispielhaft in den Figuren 2 und 3 in weiteren Details erkennbar ist. Dieses Anzeigenelement 120 kann beispielsweise ein segmentiertes Display umfassen, wie beispielsweise aus der Darstellung gemäß Figur 3 erkennbar ist. Das Anzeigenelement 120 kann beispielsweise rund ausgestaltet sein und in einem Deckel 122 des Gehäuses 118 angeordnet sein. Dieser Deckel 122 kann, wie unten erläutert, auch als aufklappbarer und/oder aufschwenkbarer Deckel 122 ausgestaltet sein.

Weiterhin umfasst das Messgerät 112 in dem in Figur 1 dargestellten Ausführungsbeispiel ein Auswahlelement 124, welches hier in Form eines zylindrischen Drehrings 126 ausgestaltet ist. Dieser Drehring ist, beispielsweise innerhalb gewisser Grenzen, um eine Achse 128 des Messgerätes 112 drehbar, während das übrige Gehäuse 118 festbleibt. Auf diese Weise lässt sich über die Drehposition des Drehringes 126 eine von mehreren Auswahlpositionen einstellen, welche durch die Drehwinkelstellung des Drehringes 126 relativ zum übrigen Gehäuse 118 gegeben ist. Die aktuell eingestellte Auswahlposition kann beispielsweise mittels einer Markierung 130 auf dem Gehäuse 118, beispielsweise dem Deckel 122, erkennbar sein. Alternativ oder zusätzlich können weitere Markierungen 130 auf dem Drehring 126 vorgesehen sein.

Weiterhin umfasst der Drehring 126 des Auswahlelementes 124 in dem dargestellten Ausfiihrungsbeispiel eine Beschriftung 132, welche als Funktionsmarkierung 134 dient. Alternativ oder zusätzlich können weitere Beschriftungen 132 und/oder weitere Funktionsmarkierungen 134 auch auf dem übrigen Gehäuse 118 des Messgerätes 112 vorgesehen sein.

Die Beschriftung 132 bezeichnet dabei verschiedene Menüpunkte und deren Inhalt. Aus der relativen Stellung der Beschriftung 132 bzw. der einzelnen Punkte dieser Beschriftung 132 zur Markierung 130 ergibt sich beispielsweise die eingestellte Auswahlposition und damit der ausgewählte Menüpunkt.

Beispielhaft sind in Figur 1 vier Menüpunkte erkennbar, welche in axialer Schriftrichtung auf dem Umfang des Drehringes 126 angebracht sind.

So ist ein erster Menüpunkt 136 vorgesehen, welcher in Figur 1 mit "AUS" markiert ist und bei dessen Auswahl das Messgerät 112 und/oder das Analysegerät 110 beispielsweise in einen ausgeschalteten Zustand oder einen Stand-by-Zustand geschaltet werden kann.

In einem zweiten Menüpunkt 138, welcher in Figur 1 mit "TEST" bezeichnet ist, kann beispielsweise eine Testroutine durchgeführt werden. In einem dritten Menüpunkt 140, welcher in Figur 1 mit "AKTUELL" bezeichnet ist, kann beispielsweise ein aktueller Messwert angezeigt werden. In einem vierten Menüpunkt 142, welcher in Figur 1 mit "Ø 7 TAGE" bezeichnet ist, kann beispielsweise ein Durchschnittswert von Messergebnissen über die letzten 7 Tage angezeigt werden.

Weitere mögliche Menüpunkte sind beispielsweise in der Darstellung gemäß Figur 2 erkennbar. So kann ein fünfter Menüpunkt 144 vorgesehen sein, welcher mit "DATUM" beschriftet ist und welcher die Einstellung eines Datums ermöglicht. Weiterhin kann ein sechster Menüpunkt 146 vorgesehen sein, welcher mit "ZEIT" bezeichnet ist und welcher die Einstellung einer Zeit ermöglicht.

Die genannten Menüpunkte 136 bis 146 stellen lediglich Beispiele dar, wie eine Menüsteuerung des Analysegerätes 110 ausgestaltet sein kann. Neben den gezeigten Kombinationen sind zahlreiche weitere Kombinationen der genannten Menüpunkte und/oder anderer Arten von Menüpunkten denkbar.

Wie aus der Beschreibung der Menüpunkte 136 bis 146 hervorgeht, benötigen einige der Menüpunkte eine Beeinflussung eines oder mehrerer Parameter durch einen Benutzer. Als Beispiel für derartige Menüpunkte, bei welchen eine Beeinflussung von Parametern durch einen Benutzer möglich sein sollte, sind die Menüpunkte 144 und 146 zu nennen, bei welchen die Einstellung eines Datums und/oder einer Zeit, beispielsweise eines Messdatums und/oder einer Messzeit und/oder einer aktuellen Zeit, möglich sein sollte.

Die oben aufgelisteten Beispiele der Menüpunkte 136 bis 146 zeigen, dass die Menüpunkte sehr unterschiedlich ausgestaltet sein können. Einige der genannten Menüpunkte, wie beispielsweise die Menüpunkte 136, 138, 140 oder 142, können beispielsweise derart ausgestaltet sein, dass diese ausschließlich Informationen an einen Benutzer bereitstellen, beispielsweise durch Anzeige auf dem Anzeigenelement 120. Alternativ oder zusätzlich können auch Menüpunkte vorgeschen sein, welche eine Einflussnahme des Benutzers erfordern, insbesondere eine Beeinflussung eines oder mehrerer Parameter. Wie oben dargestellt, kann es sich bei diesern Parametern beispielsweise um Informationen in Form von einzugebenden Daten. Variablen oder ähnliches handeln oder auch um Steuerbefehle. Ein Beispiel derartiger Menüpunkte, welche eine Beeinflussung eines oder mehrerer Parameter zumindest ermöglichen sollten, sind die Menüpunkte 144 und 146 gemäß Figur 2, also die Einstellung von Datum und/oder Zeit. Auch die Aufforderung zur Vornahme einer Messung, eine Aufforderung zur Anzeige bestimmter Informationen, eine Aufforderung zur Durchführung bestimmter Auswertungen oder ähnliches kann in dieser Kategorie der Menüpunkte subsumiert werden, welche eine Beeinflussung mindestens eines Parameters ermöglichen sollen.

Zur Beeinflussung des mindestens einen Parameters sind bei dem in den Figuren 1 und 2 gezeigten Ausführungsbeispiel Bedienelemente 148 vorgesehen. Diese Bedienelemente sind in dem dargestellten Ausführungsbeispiel in dem Auswahlelement 124 integriert und umfassen eine Vorwärtstaste 150 und eine Rückwärtstaste 152. Über die Vorwärtstaste 150, welche in den Figuren 1 und 2 mit einem nach oben gerichteten Pfeil markiert ist, können beispielsweise Parameterwerte erhöht werden oder ein Schritt in einem Funktionsmenü nach vorne vorgenommen werden. Mittels der mit einem nach unten weisenden Pfeil markierten Rückwärtstaste 152 können beispielsweise Werte vermindert werden oder Schritte in einem Auswahlmenü rückwärts vorgenommen werden. Die dargestellte Ausgestaltung der Bedienelemente 148 ist jedoch lediglich eine von zahlreichen möglichen Ausgestaltungen, wobei auch mehr Tasten als die beiden Tasten 150, 152 vorgesehen sein können, wobei auch eine andere Art von Markierung gewählt werden kann oder wobei insgesamt eine andere Ausgestaltung gewählt werden kann. Auch müssen die Bedienelemente 148 nicht fest in dem Anzeigenelement 120 integriert sein, so dass sich diese beispielsweise bei einer Drehung des Drehrings 126 mitdrehen, sondern es kann auch, alternativ oder zusätzlich und vollständig oder teilweise, eine ortsfeste Anordnung der Bedienelemente 148 gewählt werden. Verschiedene weitere Ausgestaltungen sind unten gezeigt.

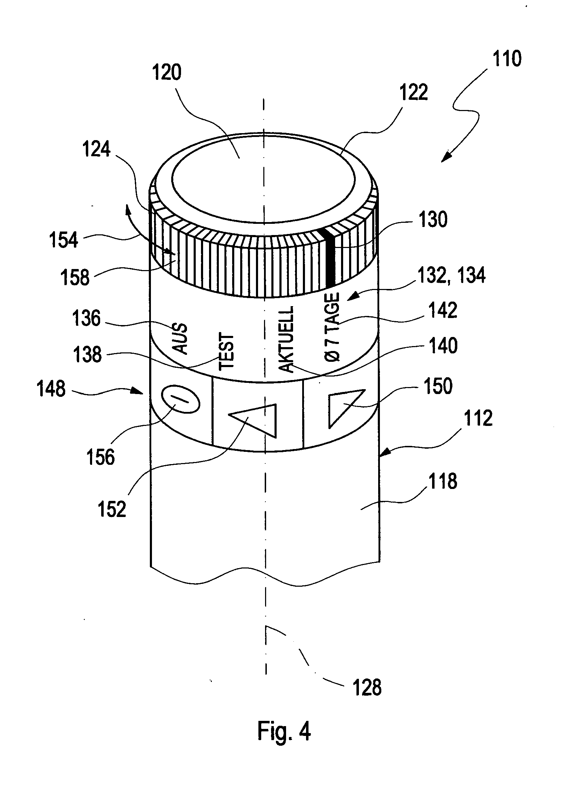

In Figur 4 ist, in zu Figur 2 analoger Darstellung, ein alternatives Ausführungsbeispiel eines Analysegerätes 110 dargstellt. Dabei ist lediglich ein Messgerät 112 gezeigt. Optional kann das Analysegerät 110 jedoch wiederum, analog zu Figur 1, ein in Figur 4 nicht dargestelltes Probengewinnungselement 114 aufweisen.

In dem Ausführungsbeispiel gemäß Figur 4 ist das Gerät 112 wiederum zylinderförmig ausgestaltet, so dass für die Ausgestaltungen zahlreicher Elemente dieses Messgerätes 112 beispielsweise auf die obige Beschreibung der Figuren 1 bis 3 verwiesen werden kann.

In den Figuren 1 bis 3 wurde angenommen, dass das Auswahlelement 124 durch den Drehring 126 gebildet wird. Alternativ zu dieser Ausgestaltung könnte jedoch auch eine Drehung des Deckels 122 relativ zum übrigen Gehäuse 118 erfolgen, so dass das Auswahlelement 124 mit dem Deckel 122 zumindest teilidentisch wäre. In diesem Fall wären beispielsweise die Beschriftung 132 und/oder die Bedienelemente 148 ganz oder teilweise ortsfest relativ zum übrigen Gehäuse 118, wohingegen der Deckel 122 um die Achse 128 gedreht würde. Das in Figur 4 gezeigte Ausführungsbeispiel ist in dieser Weise ausgestaltet. Es sei darauf hingewiesen, dass jedoch auch bei dem in Figur gezeigten Ausführungsbeispiel wahlweise wiederum auch der Deckel 122 ortsfest zum übrigen Gehäuse 118 verbleiben könnte, wohingegen unterhalb des Deckels 122, im Bereich der Beschriftung 132, wiederum ein Drehring 126 angeordnet sein könnte. Dargestellt in Figur 4 ist jedoch eine dort mit der Bezugsziffer 154 bezeichnete Drehung des Deckels 122 relativ zum übrigen Gehäuse 118, zur Auswahl einer bestimmten Auswahlposition.

Die als Funktionsmarkierung 134 fungierende Beschriftung 132 kann dabei also, wie oben dargestellt ganz oder teilweise auf dem Auswahlelement 124 angeordnet sein und/oder ganz oder teilweise auf einem übrigen, ortsfesten Bestandteil des Gehäuses 118. Die Beschriftung 132, welche beispielsweise wiederum analog zur Ausgestaltung in den Figuren 1 und 2 ausgestaltet sein kann, ist in Figur 4 lediglich symbolisch angedeutet.

Weiterhin unterscheidet sich das Ausführungsbeispiel gemäß Figur 4 in der Ausgestaltung der Bedienelemente 148. Auch diese Bedienelemente 148 sind in dem gezeigten Ausführungsbeispiel ortsfest zum übrigen Gehäuse 118 angeordnet, was beispielsweise auch Vorteile in einer elektrischen Anbindung dieser Bedienelemente 148 beinhalten kann. Alternativ ist jedoch wiederum auch eine vollständige oder teilweise Anordnung dieser Bedienelemente 148 auf dem rotierbaren Auswahlelement 124, beispielsweise im Bereich des Deckels 122, denkbar.

Auch in sonstiger Weise unterscheiden sich die Bedienelemente 148 gemäß dem Ausführungsbeispiel in Figur 4 von den Bedienelementen gemäß dem Ausführungsbeispiel in den Figuren 1 und 2. Wiederum sind eine Vorwärtstaste 150 und eine Rückwärtstaste 152 vorgesehen, beispielsweise mit den oben beschriebenen Funktionalitäten. Weiterhin ist eine Eingabetaste 156 vorgesehen, welche beispielsweise zur Bestätigung von Eingaben benutzt werden kann und/oder zur Auswahl eines bestimmten Menüs. Eine derartige Eingabetaste ist optional jedoch auch bei dem Ausführungsbeispiel gemäß den Figuren 1 und 2 integrierbar.

In dem in Figur 4 dargestellten Fall, in welchem das Auswahlelement 124 ganz oder teilweise durch einen drehbaren Deckel 122 gebildet wird, in welchem auch ein Anzeigenelement 120 integriert ist, kann der Rand dieses Deckels 122, beispielsweise durch entsprechende Griffrillen 158 greifbar ausgestaltet sein, welche in Figur 4 angedeutet sind. Auch in dem Ausführungsbeispiel gemäß den Figuren 1 bis 3 können derartige Griffrillen 158 vorgesehen sein, insbesondere wenn dort nicht die beschriebene Variante mit dem Drehring 126 verwendet wird, sondern wenn dort das Auswahlelement 124 ebenfalls durch einen drehbaren Deckel 122 realisiert wird. Wird der Deckel 122 drehbar ausgestaltet, so kann das Messgerät 112 derart eingerichtet sein, dass sich das Anzeigenelement 120 bei Drehung des Deckels 122 mit diesem mitdreht. Alternativ kann das Anzeigenelement 120 jedoch auch ganz oder teilweise drehfest verbleiben und lediglich ein Rahmen des Deckels 122 gedreht werden, welcher dann wiederum einen Drehring 126 bildet. Verschiedene Ausgestaltungen sind möglich.

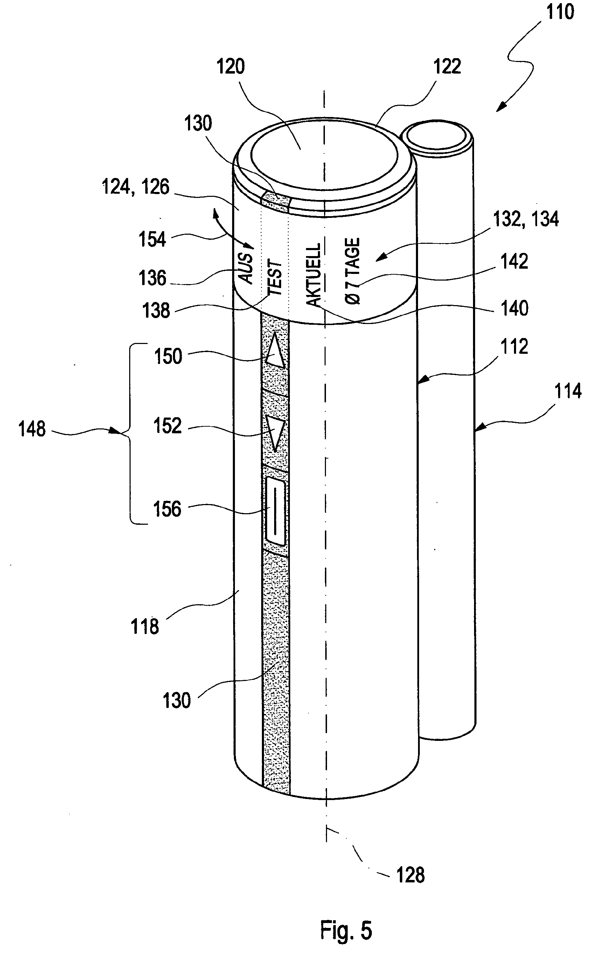

In Figur 5 ist ein weiteres, zu Figur 1 alternatives Ausführungsbeispiel eines erfindungsgemäßen Analysegerätes 110 mit einem Messgerät 112 und einem optionalen Probengewinnungselement 114 perspektivisch dargestellt. Das Probengewinnungselement 114 ist lediglich symbolisch angedeutet und kann beispielsweise analog zur Ausführungsform gemäß Figur 1 ausgestaltet sein. Das Messgerät 112 ist in dem Ausführungsbeispiel gemäß Figur 5 wiederum mit einem Gehäuse 118 versehen, welches beispielsweise wiederum im Wesentlichen zylinderförmig ausgestaltet sein kann. Für weite Teile der Ausführungsform gemäß Figur 5 kann auf die Beschreibung des Ausführungsbeispiels gemäß den Figuren 1 bis 3 verwiesen werden.

Wiederum weist das Gehäuse 118 des Messgerätes 112 einen kreisförmigen Deckel 122 auf. In diesem Ausführungsbeispiel wird angenommen, dass der Deckel 122 drehfest ausgestaltet ist, analog zur obigen Beschreibung des Ausführungsbeispiels in den Figuren 1 bis 3. Auch eine drehbare Ausgestaltung des Deckels 122 ist jedoch möglich, beispielsweise analog zur obigen Beschreibung des Ausführungsbeispiels gemäß Figur 4.

Entsprechend der gewählten Ausführungsform weist das Messgerät 112 wiederum ein Auswahlelement 124 in Form eines Drehrings 126 auf, welcher beispielsweise am oberen Ende des Gehäuses 118, unterhalb des Deckels 122, angeordnet sein kann. Auch andere Anordnungen des Drehrings 126 bzw. des Auswahlelementes 124 sind jedoch grundsätzlich möglich.

Wiederum weist, analog zur obigen Beschreibung des Ausführungsbeispiels gemäß den Figuren 1 bis 3, der Drehring 126 eine Beschriftung 132 auf, welche als Funktionsmarkierung 134 dient und welche beispielsweise analog zum Ausführungsbeispiel gemäß den Figuren 1 bis 3 ausgestaltet sein kann. Diese Beschriftung 132 ist in Figur 5 lediglich andeutungsweise gezeigt.

Das Gehäuse 118 des Messgerätes 112 weist wiederum eine Markierung 130 auf, welche in diesem Ausführungsbeispiel in Form eines in axialer Richtung verlaufenden Streifens ausgestaltet ist, der sich bis zum Deckel 122 fortsetzt und lediglich durch den Drehring 126 unterbrochen wird. Je nachdem, welcher der Menüpunkte der Funktionsmarkierung 134 linear zu dieser streifenförmigen Markierung 130 durch eine entsprechende Drehung 154 ausgerichtet wird, wird ein bestimmter Menüpunkt eines Funktionsmenüs ausgewählt.

Wiederum weist das Messgerät 112 weiterhin Bedienelemente 148 auf. Dabei zeigt Figur 5 eine optionale Ausführungsform, bei welcher diese Bedienelemente 148 ganz oder teilweise in die Markierung 130 integriert sind, so dass intuitiv unmittelbar klar ist, dass sich diese Bedienelemente auf den jeweils ausgewählten Menüpunkt beziehen. Die Bedienelemente 148 können wiederum beispielsweise eine Vorwärtstaste 150, eine Rückwärtstaste 152 und eine Eingabetaste 156 umfassen. Auch andere Ausgestaltungen sind jedoch möglich, beispielsweise eine Ausgestaltung mit zwei Tasten 150, 152, beispielsweise Figur 2.

In Figur 6 ist in einer Schnittdarstellung ein möglicher Innenaufbau eines Messgerätes 112 gezeigt, beispielsweise gemäß einem oder mehrerer der Ausführungsbeispiele gemäß den Figuren 1 bis 5. Es sei darauf hingewiesen, dass auch ein anderer Aufbau möglich ist. Für weite Teile der Ausführungsform kann dementsprechend beispielsweise auf die Beschreibung der obigen Figuren verwiesen werden. Lediglich beispielhaft ist ein Messgerät 112 gezeigt, bei welchem das Auswahlelement 124 ganz oder teilweise im Deckel 122 integriert ist. Alternativ sind jedoch auch die Ausführungsformen mit einem Drehring möglich, beispielsweise gemäß Figur 1 oder gemäß Figur 5. Die Bedienelemente 148 sind in Figur 6 nicht dargestellt.

Bei dem in Figur 6 gezeigten, beispielsweise wiederum zylinderförmig ausgestalteten Gehäuse 118 des Messgerätes 112 sind verschiedene Bauelemente entlang der Achse 128 des Messgerätes 112 angeordnet. So kann das Messgerät 112 beispielsweise eine Messeinheit 160 umfassen, welche in diesem Ausführungsbeispiel unterhalb des Deckels 122 angeordnet ist. Auch eine andere Anordnung ist jedoch grundsätzlich möglich, beispielsweise an einem unteren Ende des Gehäuses 118, dem Deckel 122 gegenüberliegend.

Die Messeinheit 160 ist optional als Messeinheit ausgestaltet, welche auf der Verwendung eines oder mehrerer Testelemente 162 basiert. Dabei sind Ausführungsformen möglich, bei welchen diese Testelemente 162 von außen in das Messgerät 112 eingegeben werden. In Figur 6 ist eine Option gezeigt, bei welcher mehrere Testelemente 162 in einem Magazin, beispielsweise einem zylinderförmigen Trommelmagazin, im Inneren des Gehäuses 118 angeordnet sind und nacheinander an die Messeinheit 160 bereitgestellt werden können. Verschiedene andere Ausgestaltungen sind möglich, beispielsweise die Verwendung von Stapelmagazinen, Bandmagazinen, Testbändern oder ähnlichem. Das Trommelmagazin ist in Figur 6 mit der Bezugziffer 164 bezeichnet.

Das Trommelmagazin 164 ist zur Bereitstellung von Testelementen 162 optional um die Achse 128 oder um eine zu dieser Achse 128 parallele Achse drehbar. Um diese Drehbarkeit zu gewährleisten umfasst das Messgerät 112 einen Antrieb 166, welcher beispielsweise einen oder mehrere Motoren sowie ggf. ein geeignetes Getriebe umfassen kann und welcher eine Drehung der Trommelmagazins 164 bewerkstelligen kann.

Schließlich umfasst das Messgerät 112 ein oder mehrere Energiespeicher 168, beispielsweise eine oder mehrere Batterien und/oder Akkumulatoren. Diese können elektrische Energie beispielsweise an die Messeinheit 160 und/oder an den Antrieb 166 bereitstellen.

Das Messgerät 112 umfasst weiterhin eine oder mehrere Steuerungen 170. Diese Steuerung 170 kann beispielsweise ganz oder teilweise mit der Messeinheit 160 zusammengefasst sein. Alternativ oder zusätzlich ist jedoch auch eine von der Messeinheit 160 getrennte Steuerung 170 realisierbar, beispielsweise eine dezentrale Steuerung. Eine derartige Steuerung 170 kann beispielsweise eingerichtet sein, um die oben dargestellte Menüsteuerung vorzunehmen und entsprechend beispielsweise eine Steuerung der Messungen vorzunehmen, eine Datenspeicherung und Datenverwaltung, eine Datenauswertung oder ähnliches vorzunehmen. Verschiedene Ausgestaltungen sind denkbar.

In Figur 7 ist schließlich ein Ausführungsbeispiel eines Analysegerätes 110 mit einem Messgerät 112 gezeigt, welches einen schwenkbaren Deckel 122 umfasst. Das Messgerät 112 kann dabei beispielsweise analog zu den oben beschriebenen Ausführungsformen ausgestaltet sein. Im Folgenden wird angenommen, dass das Analysegerät analog zum Ausführungsbeispiel gemäß den Figuren 1 bis 3 ausgestaltet ist, wobei ein Auswahlelement 124 in Form eines Drehringes 126 vorgesehen ist.

Alternativ oder zusätzlich kann jedoch auch wiederum der Deckel 122 ganz oder teilweise als drehbares Auswahlelement 124 ausgestaltet sein. Bezüglich weiterer möglicher Ausgestaltungen des Messgerätes 112 kann dementsprechend auf die obige Beschreibung verwiesen werden. Der Deckel 122, welcher wiederum beispielsweise das Anzeigenelement 120 umfassen kann, ist dabei bei dem in Figur 7 gezeigten Ausführungsbeispiel um eine Drehachse 172 drehbar gelagert, so dass sich der Deckel 122 aus der zur Achse 128 konzentrischen Ausrichtung lenken lässt. In dieser in Figur 7 dargestellten ausgeschwenkten Stellung wird eine symbolisch mit der Bezugsziffer 174 bezeichnete Applikationsposition im Inneren des Messgerätes 112 freigegeben, in welcher beispielsweise ein Teststreifen 162 aus einem Ausgabeschlitz 176 soweit ausgeschoben werden kann, dass eine Aufgabe einer Blutprobe und/oder einer anderen flüssigen Probe erfolgen kann. Auch eine Messung kann in dieser Position optional erfolgen. Nach der Messung kann das Testelement 162 wieder in das Magazin, beispielsweise das Trommelmagazin 164, remagaziniert oder kann auf andere Weise entsorgt, beispielsweise ausgegeben werden.

Dabei kann das Messgerät 112 ausgestaltet sein, dass auch während des Aufklappens des Deckels 122 oder in aufgeklappter Stellung eine Bedienung des Auswahlelementes 124 möglich ist. Alternativ kann die Ausgestaltung jedoch auch derart erfolgen, dass erst wieder im geschlossenen Zustand eine derartige Bedienung möglich ist. Verschiedene Ausführungsformen sind denkbar.

### Bezugzeichenliste

- 110: Analysegerät
- 112: Messgerät
- 114: Probengewinnungselement
- 116: Einstellknopf
- 118: Gehäuse
- 120: Anzeigenelement
- 122: Deckel
- 124: Auswahlelement
- 126: Drehring
- 128: Achse
- 130: Markierung
- 132: Beschriftung
- 134: Funktionsmarkierung
- 136: erster Menüpunkt
- 138: zweiter Menüpunkt
- 140: dritter Menüpunkt
- 142: vierter Menüpunkt
- 144: fünfter Menüpunkt
- 146: sechster Menüpunkt
- 148: Bedienelemente
- 150: Vorwärtstaste
- 152: Rückwärtstate
- 154: Drehung
- 156: Eingabetaste
- 158: Griffrillen
- 160: Messeinheit
- 162: Testelement
- 164: Trommelmagazin
- 166: Antrieb
- 168: Energiespeicher
- 170: Steuerung
- 172: Drehachse
- 174: Applikationsposition
- 176: Ausgabeschlitz

## Patentansprüche

1. Analysegerät (110) zum Nachweis eines Analyten in einer flüssigen Probe, insbesondere zum Nachweis von Glucose in einer Körperflüssigkeit, umfassend mindestens eine Steuerung (170), wobei die Steuerung (170) eingerichtet ist, um ein Funktionsmenü mit mindestens zwei Menüpunkten bereitzustellen, weiterhin umfassend mindestens ein mechanisch verstellbares Auswahlelement (124), wobei das Auswahlelement (124) durch einen Benutzer in mindestens zwei verschiedene Auswahlpositionen einstellbar ist, wobei das Auswahlelement (124) eingerichtet ist, um nach dem Einstellen in der eingestellten Auswahlposition zu verbleiben, wobei das Analysegerät (110) ein Gehäuse (118) aufweist, wobei das Gehäuse (118) und/oder das Auswahlelement (124) mindestens eine Funktionsmarkierung (134) mit mindestens einer Beschriftung (132) des Auswahlelements (124) und/oder des Gehäuses (118) aufweist, wobei die Funktionsmarkierung (134) mindestens einen der Menüpunkte und/oder mindestens einen Inhalt mindestens eines der Menüpunkte bezeichnet, wobei die Beschriftung (132) eingerichtet ist, um unmittelbar und unabhängig von einer elektrischen Funktionalität des Analysegeräts (110) eine Menüsteuerung zu erläutern, wobei die Steuerung (170) eingerichtet ist, um entsprechend der eingestellten Auswahlposition einen Menüpunkt auszuwählen, wobei mit dem Auswahlelement (124) ein direktes Springen zu vorgegebenen Menüpunkten ermöglicht wird, wobei durch einen Benutzer unmittelbar über das Auswahlelement ein bestimmter Menüpunkt auswählbar ist, wobei das Analysegerät (110) weiterhin mindestens ein Bedienelement (148) aufweist, wobei in dem ausgewählten Menüpunkt mittels des Bedienelements (148) mindestens ein Parameter durch den Benutzer beeinflussbar ist.

2. Analysegerät (110) nach dem vorhergehenden Anspruch, wobei das Gehäuse (118) eine für den Benutzer wahrnehmbare Markierung (130) aufweist, wobei das Auswahlelement (124) relativ zu der Markierung (130) in seiner Position und/oder Orientierung verstellbar ist.

3. Analysegerät (110) nach dem vorhergehenden Anspruch 2, wobei das Bedienelement (148) und die Markierung (130) zumindest teilweise zusammengefasst sind.

4. Analysegerät (110) nach einem der vorhergehenden Ansprüche, wobei die Funktionsmarkierung (134) eingerichtet ist, um mittels des Bedienelements (148) beeinflussbare Parameter des durch die Auswahlposition ausgewählten Menüpunkts zumindest teilweise anzuzeigen.

5. Analysegerät (110) nach einem der vorhergehenden Ansprüche, wobei das Auswahlelement (124) mindestens einen Drehschalter und/oder einen Schiebeschalter umfasst.

6. Analysegerät (110) nach einem der vorhergehenden Ansprüche, wobei das Gehäuse (118) zumindest teilweise im Wesentlichen zylinderförmig ausgestaltet ist, wobei das Auswahlelement (124) mindestens einen das Gehäuse (118) zumindest teilweise umschließenden Drehring (126) umfasst, wobei der Drehring (126) zur Einstellung der Auswahlposition relativ zum übrigen Gehäuse (118) verdrehbar ist.

7. Analysegerät (110) nach einem der vorhergehenden Ansprüche, wobei das Analysegerät (110) einen drehbaren Deckel (122) umfasst, wobei das Auswahlelement (124) ganz oder teilweise in den drehbaren Deckel (122) integriert ist.

8. Analysegerät (110) nach einem der vorhergehenden Ansprüche, wobei das Bedienelement (148) zumindest teilweise in dem Auswahlelement (124) integriert ist.

9. Analysegerät (110) nach einem der vorhergehenden Ansprüche, wobei nicht mehr als drei Bedienelemente (148) vorgesehen sind.

10. Analysegerät (110) nach einem der vorhergehenden Ansprüche, weiterhin umfassend ein Anzeigenelement (120) zur Anzeige mindestens einer veränderlichen Information.

11. Analysegerät (110) nach dem vorhergehenden Anspruch 10, wobei das Anzeigenelement (120) mindestens ein segmentiertes Display umfasst.

12. Analysegerät (110) nach einem der beiden vorhergehenden Ansprüche 10 oder 11 wobei das Anzeigenelement (120) zumindest teilweise in einem Deckel (122) des Gehäuses (118) des Analysegeräts (110) integriert ist, insbesondere einem Deckel (122) eines zylindrischen Gehäuses (118) des Analysegeräts (110).

13. Analysegerät (110) nach einem der vorhergehenden Ansprüche, wobei das Analysegerät (110) einen Deckel (122) umfasst, wobei der Deckel (122) aufschwenkbar und/oder aufklappbar ausgestaltet ist, um mindestens ein innenliegendes Element des Analysegeräts (110), insbesondere eine Applikationsposition (174) zur Aufgabe der flüssigen Probe und/oder ein Testelement (162) freizugeben.

14. Analysegerät (110) nach einem der vorhergehenden Ansprüche, umfassend weiterhin mindestens ein Probengewinnungselement (114), welches eingerichtet ist, um eine Hautpartie eines Benutzers zu perforieren.

15. Analysegerät (110) nach dem vorhergehenden Anspruch 14, wobei das Probengewinnungselement (114) von dem übrigen Analysegerät (110) reversibel abkoppelbar und separat verwendbar ist.

## Claims

1. Analysis device (110) for detecting an analyte in a liquid sample, in particular for detecting glucose in a bodily fluid, comprising at least one control element (170), wherein the control element (170) is configured to provide a function menu with at least two menu items, and further comprising at least one mechanically adjustable selector element (124), wherein the selector element (124) can be adjusted by a user to at least two different selection positions, wherein the selector element (124) is configured to remain in the adjusted selection position after being adjusted, wherein the analysis device (110) has a housing (118), wherein the housing (118) and/or the selector element (124) has at least one function marking (134) with at least one inscription (132) of the selector element (124) and/or of the housing (118), wherein the function marking (134) designates at least one of the menu items and/or at least a content of at least one of the menu items, wherein the inscription (132) is designed to explain a menu control directly and independently of an electrical functionality of the analysis device (110), wherein the control element (170) is configured to select a menu item corresponding to the adjusted selection position, wherein the selector element (124) allows the user to jump directly to predefined menu items, wherein a certain menu item can be selected by a user directly via the selector element, wherein the analysis device (110) further comprises at least one operating element (148), wherein at least one parameter in the selected menu item can be influenced by the user via the operating element (148).

2. Analysis device (110) according to the preceding claim, wherein the housing (118) has a marking (130) perceptible to the user, wherein the selector element (124) can be adjusted in position and/or orientation relative to the marking (130).

3. Analysis device (110) according to the preceding Claim 2, wherein the operating element (148) and the marking (130) are at least partially combined.

4. Analysis device (110) according to one of the preceding claims, wherein the function marking (134) is configured to at least partially indicate parameters, which can be influenced by means of the operating element (148), of the menu item selected by the selection position.

5. Analysis device (110) according to one of the preceding claims, wherein the selector element (124) comprises at least one rotary switch and/or one slide switch.

6. Analysis device (110) according to one of the preceding claims, wherein the housing (118) is at least in part substantially cylindrical, wherein the selector element (124) comprises at least one rotary ring (126) at least partially surrounding the housing (118), wherein the rotary ring (126) can be rotated relative to the rest of the housing (118) in order to adjust the selection position.

7. Analysis device (110) according to one of the preceding claims, wherein the analysis device (110) comprises a rotatable lid (122), wherein the selector element (124) is completely or partially integrated in the rotatable lid (122).

8. Analysis device (110) according to one of the preceding claims, wherein the operating element (148) is at least partially integrated in the selector element (124).

9. Analysis device (110) according to one of the preceding claims, wherein no more than three operating elements (148) are provided.

10. Analysis device (110) according to one of the preceding claims, further comprising an indicator element (120) for displaying at least one variable item of information.

11. Analysis device (110) according to the preceding Claim 10, wherein the indicator element (120) comprises at least one segmented display.

12. Analysis device (110) according to one of the two preceding Claims 10 and 11, wherein the indicator element (120) is at least partially integrated in a lid (122) of the housing (118) of the analysis device (110), particularly a lid (122) of a cylindrical housing (118) of the analysis device (110).

13. Analysis device (110) according to one of the preceding claims, wherein the analysis device (110) comprises a lid (122), wherein the lid (122) is designed such that it can be pivoted up and/or flipped up in order to free at least one internal element of the analysis device (110), in particular an application position (174) for placing the liquid sample on and/or a test element (162).

14. Analysis device (110) according to one of the preceding claims, further comprising at least one sample-collecting element (114), which is designed to puncture an area of skin of a user.

15. Analysis device (110) according to the preceding Claim 14, wherein the sample-collecting element (114) can be reversibly uncoupled from the rest of the analysis device (110) and used separately.

## Revendications

1. Appareil d'analyse (110) destiné à détecter un analyte dans un échantillon liquide, en particulier à détecter le glucose dans un liquide corporel,
comprenant au moins une commande (170), la commande (170) étant conçue pour préparer un menu de fonctions qui présente au moins deux points de menu,
et comprenant en outre au moins un élément de sélection (124) ajustable mécaniquement, l'élément de sélection (124) pouvant être placé en au moins deux positions de sélection différentes par un utilisateur,
l'élément de sélection (124) étant conçu pour rester dans la position de sélection qui a été établie,
l'appareil d'analyse (110) présentant un boîtier (118), le boîtier (118) et/ou l'élément de sélection (124) présentant au moins un repère de fonction (134) qui présente au moins une inscription (132) sur l'élément de sélection (124) et/ou sur le boîtier (118),
le repère de fonction (134) désignant au moins l'un des points du menu ou au moins un contenu d'au moins un des points du menu, l'inscription (132) étant conçue pour expliquer une commande de menu, directement et indépendamment d'une fonctionnalité électrique de l'appareil d'analyse (110),
la commande (170) étant conçue pour sélectionner un point du menu en fonction de la position de sélection qui a été établie, l'élément de sélection (124) permettant de sauter directement à des points prédéterminés du menu,
l'élément de sélection permettant à un utilisateur de sélectionner directement un point défini du menu,
l'appareil d'analyse (110) présentant en outre au moins un élément de commande (148), l'élément de commande (148) permettant à l'utilisateur d'agir sur au moins un paramètre dans le point de menu sélectionné.

2. Appareil d'analyse (110) selon la revendication précédente, dans lequel le boîtier (118) présente un repère (130) détectable par l'utilisateur, la position et/ou l'orientation de l'élément de sélection (124) par rapport au repère (130) pouvant être déplacées.

3. Appareil d'analyse (110) selon la revendication 2 qui précède, dans lequel l'élément de commande (148) et le repère (130) sont au moins partiellement rassemblés.

4. Appareil d'analyse (110) selon l'une des revendications précédentes, dans lequel le repère de fonction (134) est conçu pour afficher au moins partiellement le paramètre du point du menu sélectionné par la position de sélection et sur lequel l'élément de commande (148) permet d'agir.

5. Appareil d'analyse (110) selon l'une des revendications précédentes, dans lequel l'élément de sélection (124) comporte au moins un commutateur rotatif et/ou un commutateur coulissant.

6. Appareil d'analyse (110) selon l'une des revendications précédentes, dans lequel au moins une partie du boîtier (118) a une configuration essentiellement cylindrique, l'élément de sélection (124) comprenant au moins un anneau rotatif (126) qui entoure au moins une partie du boîtier (118), l'anneau rotatif (126) pouvant être tourné par rapport au reste du boîtier (118) pour établir la position de sélection.

7. Appareil d'analyse (110) selon l'une des revendications précédentes, dans lequel l'appareil d'analyse (110) comporte un couvercle rotatif (122), l'élément de sélection (124) étant complètement ou partiellement intégré dans le couvercle rotatif (122).

8. Appareil d'analyse (110) selon l'une des revendications précédentes, dans lequel au moins une partie de l'élément de commande (148) est intégrée dans l'élément de sélection (124).

9. Appareil d'analyse (110) selon l'une des revendications précédentes, qui ne présente pas plus de trois éléments de commande (148).

10. Appareil d'analyse (110) selon l'une des revendications précédentes, comprenant en outre un élément d'affichage (120) qui affiche au moins une information modifiable.

11. Appareil d'analyse (110) selon la revendication 10 qui précède, dans lequel l'élément d'affichage (120) comporte au moins un affichage segmenté.

12. Appareil d'analyse (110) selon l'une des deux revendications 10 et 11 qui précèdent, dans lequel au moins une partie de l'élément d'affichage (120) est intégrée dans un couvercle (122) du boîtier (118) de l'appareil d'analyse (110) et en particulier dans un couvercle (122) d'un boîtier cylindrique (118) de l'appareil d'analyse (110).

13. Appareil d'analyse (110) selon l'une des revendications précédentes, l'appareil d'analyse (110) comportant un couvercle (122), le couvercle (122) étant configuré de manière à pouvoir pivoter et/ou être rabattu pour libérer au moins un élément intérieur de l'appareil d'analyse (110), en particulier une position d'application (174) qui permet de placer l'échantillon liquide et/ou un élément de test (162).

14. Appareil d'analyse (110) selon l'une des revendications précédentes, comprenant en outre au moins un élément (114) de prélèvement d'échantillon conçu pour perforer une partie de la peau d'un utilisateur.

15. Appareil d'analyse (110) selon la revendication 14 qui précède, dans lequel l'élément (114) de prélèvement d'échantillon peut être découplé de manière réversible du reste de l'appareil d'analyse (110) et peut être utilisé séparément.
